**Europäisches Patentamt**

**European Patent Office**

**Office européen des brevets**

(19)

(11) Publication number: **0 105 141**
**A2**

(12) # EUROPEAN PATENT APPLICATION

(21) Application number: **83107919.9**

(22) Date of filing: **10.08.83**

(51) Int. Cl.³: **C 12 N 15/00,** C 12 P 21/02,
C 12 N 5/00, A 61 K 39/29,
G 01 N 33/54

(30) Priority: **09.09.82 GB 8225685**

(43) Date of publication of application: **11.04.84**
**Bulletin 84/15**

(84) Designated Contracting States: **AT BE CH DE FR GB IT
LI LU NL SE**

(71) Applicant: **Max-Planck-Gesellschaft zur Förderung der
Wissenschaften e.V., Bunsenstrasse 10,
D-3400 Göttingen (DE)**

(72) Inventor: **Hofschneider, Peter Hans, Prof. Dr., Nördliche
Auffahrtsallee 65, D-8000 Munich 19 (DE)**
Inventor: **Stratowa, Christial, Dr., Planeggerstrasse 135,
D-8000 München 60 (DE)**
Inventor: **Döhmer, Johannes, Dr., Klopferspitz 12,
D-8033 Martinsried (DE)**

(74) Representative: **Vossius Vossius Tauchner Heunemann
Rauh, Siebertstrasse 4 P.O. Box 86 07 67,
D-8000 München 86 (DE)**

(54) Recombinant DNA molecules, process for their production, their use for production of hepatitis B surface antigen (HBsAg) and pharmaceutical compositions containing this HBsAg.

(57) Described are a) recombinant DNA molecules containing a DNA fragment from Hepatitis B Virus comprising the structural gene of the Hepatitis B surface antigen; b) recombinant cloning vehicles containing said recombinant DNA molecules; c) microbial hosts containing said recombinant cloning vehicles; d) genomes or parts thereof of animal viruses as eukaryotic vectors containing said recombinant DNA molecules or recombinant cloning vehicles for selection of vertebrate cells and expression of at least one Hepatits B surface antigen in the selected cells; e) established vertebrate cell lines continuously producing in a nutrient medium large amounts of at least one Hepatitis B surface antigen which is released into the nutrient medium; f) compositions containing at least one Hepatitis B surface antigen as produced by said cell lines for stimulating the production of antibodies in humans to Hepatitis B Virus infection or for detecting Hepatitis B Virus infection or antibodies to Hepatitis B Virus.

Our·Ref.: S 576EP

MAX PLANCK GESELLSCHAFT ZUR FÖRDERUNG DER WISSENSCHAFTEN e.V.
3400 Göttingen

RECOMBINANT DNA MOLECULES, PROCESS FOR THEIR PRODUCTION, THEIR USE FOR PRODUCTION OF HEPATITIS B SURFACE ANTIGEN (HBsAg) AND PHARMACEUTICAL COMPOSITIONS CONTAINING THIS HBsAg

BACKGROUND OF THE INVENTION; FIELD OF THE INVENTION

This invention relates to the field of recombinant DNA technology and its use for the production of a polypeptide displaying Hepatitis B surface antigenicity. The recombinant DNA molecules disclosed here contain at least one viral genome or a part thereof functioning as eukaryotic vector for introducing into cultivated vertebrate cells a genome fragment of Hepatitis B Virus (HBV) encoding the surface antigen of HBV (HBsAg). These recombinant DNA molecules may contain also a bacterial plasmid or parts thereof for cloning and propagating in a suitable host. HBsAg stimulates the formation of antibodies in humans and therefore can be used as a vaccine against HBV and for the detection of HBV infection in humans.

This invention more specifically relates to (1) the construction of eukaryotic vectors containing the HBsAg gene, (2) their use for introduction of the HBsAg gene into cultivated animal cells, (3) the selection of cells producing the HBsAg, (4) the establishment of these HBsAg producing cells as cell lines, (5) the isolation of the HBsAg formed, and (6) the immunological and physical characterization of the surface antigen produced from these cell cultures in comparison to the Hepatitis B Virus surface antigen isolated from human serum. Furthermore, the invention relates to pharmaceutical compositions, comprising a polypeptide displaying Hepatitis B surface antigenicity

0105141

produced by cultivated animal cells and the use of this polypeptide for the detection of HBV infection in humans.

BACKGROUND ART

Hepatitis B or serum hepatitis (HBV) is a widespread viral disease. Vaccination is the only protection against HBV. Recently, several companies (for example Merck, Sharp and Dohme, U.S.A. and the Pasteur Institute, France) have introduced a vaccine against HBV. The vaccine contains as antigen a viral protein located on the envelope of the viral particle (HBsAg). Although the surface antigen itself is not pathogenic, it stimulates the production of antibodies against HBV particles in human donors. The only commercial source available for HBsAg is the blood of HBV-infected humans. Production of a safe vaccine from human serum is a cumbersome, time consuming process and therefore is relatively expensive; see e.g. "Hepatitis B Vaccine", Elsevier/NorthHolland Biomedical Press, P.Maupas and P.Guesry ed Amsterdam, New York, Oxford, (1981). Furthermore, quantities of the vaccine are limited. For these reasons the vaccine is available preferentially to groups exposed to a high risk of infection, e.g. physicians, nurses, dialysis patients, relatives of chronic carriers, newborns of mothers positive for HBsAg. For large scale vaccination, especially in third world countries where Hepatitis B is endemic, it is of great importance to have a relatively inexpensive and essentially unlimited source of HBsAg. In view of the danger of the aquired immune deficiency syndrome (AIDS), it is desirable to avoid the use of human serum as a source for HBsAg (R.Walgate, Nature, vol.304, 297 (1983)). Therefore, in order to overcome the shortage of HBsAg and to replace the only commercial now available - HBV infectious human serum - with a saferone, it seems necessary to employ methods in gene technology.

Recent advances in molecular biology have made it possible to clone and to sequence the complete genome of HBV. In addition, the coding region for the viral proteins has been identified (Published European patent applications No. 13828, 20251, and 38765; F.Galibert et al., Nature, vol.281, 646-650 (1979); M.Pasek et al., Nature, vol.282, 575-579 (1979); P.Valenzuela et al., Nature, vol.280, 815-819 (1979))

Following these advances, several host systems have been tested for expression of the viral genome, in particular that part encoding the HBV surface antigen (HBsAg).

When bacteria were used as host organisms, only at best low level production of HBsAg related immunogenic material was achieved even with powerful bacterial promoters attached to the HBV genome (C.J.Burrell, et al., Nature, vol.279 43-47, (1979); J.C.Edman, et al., Nature, vol.291, 503-506 (1981); P.MacKay, et al., Proc.Natl. Acad.Sci. U.S.A. vol.78, 4510-4514 (1981)). Although expression of various eukaryotic genes in bacteria like Escherichia coli or Bacillus subtilis have been reported, there are several reasons why it will be of advantage to use higher organisms for this application of recombinant DNA technology. (1) Bacteria can not provide certain processing mechanisms unique for eukaryotic cells, such as splicing out of introns or proteolytic cleavage of precursor proteins. (2) Bacteria do not glycosylate, phosphorylate or methylate proteins. These post-translational modifications may be important for the immunogenicity of proteins of this type. (3) Bacteria do not have a secretion mechanism which recognizes the so-called signal peptide in eukaryotic gene products. (4) Eukaryotic expression signals like eukaryotic promoters do not function in bacteria and therefore have to be replaced by prokaryotic promoters. Having to modify a eukaryotic gene raises the possibility of destroying other signals necessary for proper expression. Other eukaryotic signals like that for polyadenylation have no equivalent in bacteria at all. (5) Codon usage can be different in eukaryotic and prokaryotic organisms. Therefore, eukaryotic genes may be translated inefficiently in prokaryotic organisms. (6) Certain bacterial cell components (for example lipopolysaccharides) are highly toxic to humans and pose a serious problem in purification.

Given that the bacterial environment does not seem suitable for proper expression of many, perhaps most eukaryotic or viral genes, it will be important to develop and utilize eukaryotic gene expression systems based on higher organisms.

In fact, because of the disadvantages of using bacteria, yeast have been examined as an alternative host system (P.Valenzuela, et al., Nature, vol.298, 347-350 (1982)). The HBsAg coding sequence has been linked to the yeast alcohol dehydrogenase I promoter. Yeast transformed with this recombinant DNA molecule synthesize and accumulate HBsAg in substantial amounts of 2 to 5 ug per 200 ml yeast culture. Nevertheless, there are disadvantages in using this system. First, since the HBsAg is not released from yeast cells into the culture medium it has to be isolated from the yeast cellular extract. This requires extensive purification procedures. A host organism which secretes the HBsAg would be a more economical source because of continuous production and "easy access" to the HBsAg for isolation and purification. A second disadvantage is that the HBsAg made in the yeast cells is not glycosylated, i.e., the band corresponding to glycosylated HBsAg is missing in sodium dodecylsulfate - polyacrylamide electrophoretic gels. This HBsAg may have a immunogenic potential lower than the glycosylated form. Because of its increased stability a glycosylated peptide can increase the level and duration of immunity.

Several attempts have been made to establish mammalian cell lines that produce HBsAg. These lines are derived from human hepatocellular carcinomas (J.J.Alexander, et al., S.Afr.med.J. vol.50, 2124-2128 (1976); D.P.Aden, et al., Nature, vol.282, 615-616 (1979) ) as well as from cells transfected with cloned HBV DNA (S.Z.Hirschman, et al., Proc.Natl.Acad.Sci.U.S.A. vol.77, 5507-5511 (1980); M.F.Dubois, et al., Proc.Natl.Acad.Sci.U.S.A., vol.77, 4549-4553 (1980); J.K.Christman, et al., Proc.Natl.Acad. Sci.U.S.A., vol. 79, 1815-1819 (1982)). Monkey kidney cells have been infected with a Simian Virus 40 recombinant carrying 40% of the HBV genome (A.M.Moriarty, et al., Proc.Natl.Acad. Sci.U.S.A., vol.78, 2606-2610 (1981)). Head-to-tail tandems of the HBV genome have been introduced into mouse fibroblasts by cotransfection with selectable markers, e.g., the herpes simplex virus thymidine kinase gene (M.F.Dubois, et al., Proc.Natl.Acad. Sci.U.S.A., vol.77, 4549-4553 (1980)) and the methotrexate-resistance dihydrofolate reductase gene (J.K.Christman, et al., Proc.Natl.Acad.Sci.U.S.A., vol.79, 1815-1819 (1982)).

SUMMARY OF THE INVENTION

According to the present invention the genome of the RNA tumorvirus Moloney-Mouse-Sarcoma-Virus (MSV) and/or the DNA virus Bovine Papilloma Virus (BPV) as well as modified constructions of these genomes are used as eukaryotic vectors for the genomic part of the HBV encoding the HBsAg in order to establish cell lines producing an immunogenic Hepatitis B Virus surface antigen secreted into the nutrient medium of the cell culture in high yield. This antigen is useful for active vaccination of humans. The term "modified constructions" of MSV and BPV genomes denotes for example, introduction of additional eukaryotic expression signals and omission of genomic fragments, e.g. the transforming region of MSV and BPV.

The process for production of HBsAg disclosed here is distinguished from the prior art processes mentioned above in that none of the prior art processes uses MSV or BPV as eukaryotic vectors for the HBV genome or parts thereof, and in that vertebrate cell lines preferentially of mammalian origin are used which contain such vectors and which secrete into a nutrient medium large amounts of at least one Hepatitis B surface antigen.
Finally, a preferred embodiment of the present invention is the utilization of the natural expression signals of the gene coding for HBsAg.

A further example for a preferred natural eukaryotic expression signal is the metallothioneine signal from mouse cells; see R.D.Palmiter, et al., Cell, vol.29, 701 - 716 (1982); R.D.Palmiter, et al., Nature, vol.300, 611 - 615 (1982). According to the present invention any vertebrate cell line can be used which is capable of the replication and/or expression of a compatible inventive recombinant DNA molecule, e.g., NIH 3T3, LTK⁻, Vero, WI38, BHK, CHO, and HeLa cell lines.

In the description of this invention, the following terms are used:

Integrated proviral genome: Viral RNA which has been converted into circular double stranded DNA, then integrated by a specific process into the genome of the host cell.

Large terminal repeat (LTR): Sequences unique to RNA-tumor-viruses organized like DNA structures known for jumping DNA elements and containing both eukaryotic promoter and additional expression signals for example the so-called "enhancer sequence" or the glucocorticoid receptor site which can enhance the expression of a nearby gene by a factor of 5 to 50.

Expression signal: A DNA sequence, e.g. enhancer sequence, glucocorticoid receptor site or metallothioneine promoter facilitating and/or enhancing (controlling) the transcription of a structural gene.

Enhancer sequence: A DNA sequence preceding eukaryotic promoters enhancing the transcription of a structural gene by a not yet known mechanism.

Glucocorticoid receptor site (GRS): A DNA sequence recognized by the cytoplasmic receptor which is activated by a glucocorticoid hormone , e.g. dexamethasone. Binding of the activated receptor causes increased transcription of the structural gene.

Signal peptide (leader sequence): A hydrophobic aminoacid sequence causing secretion of the gene product.

Origin of replication: A DNA sequence serving as a recognition signal for the DNA polymerase to multiply the given genome.

Polyadenylation signal: A DNA sequence causing the prolongation of a given RNA by a number of adenine bases which results in e.g. stabilization of the mRNA by an as yet unknown mechanism.

Cloning vehicle: A DNA molecule capable of multiplying itself and DNA linked to it within a microbial host.

Structural gene: A DNA sequence which encodes through its template, i.e., messenger RNA, a sequence of amino acids characteristic of a specific polypeptide.

Subgenomic fragment: A DNA sequence comprising a part of a total DNA sequence.

Transforming region: A structural gene (oncogene) encoding a gene product which can transform a cell into a neoplastic cell.

Promoter: A DNA sequence necessary for the expression of a structural gene.

NIH 3T3 mouse fibroblasts: An established mouse cell line showing some characteristics of normal cells, like density growth dependency (G.Todaro, and H.Green, J.Cell Biol. vol.17, 299 - 313 (1963)).

LTK⁻ mouse fibroblasts: An established mouse cell line derived from strain L (W.R.Earle, J.Nat.Cancer Inst. vol.4, 165 (1943)) deficient in thymidine kinase activity (S.Kit, et al., Exp.Cell Res. vol.31, 297 - 312 (1963)).

Vero cell line: An established cell line from African Green Monkey kidney cells (Y.Yasumura, and Y.Kawakita, Nippon Rinsho vol.21, 1209 (1963)).

Microbial host: A prokaryotic or eukaryotic transformable microorganism preferably Escherichia coli suitable to

0105141

propagate plasmids.

BRIEF DESCRIPTION OF THE DRAWINGS

FIG.1 illustrating the strategy for construction of the novel recombinant DNA molecules.

FIG.2 shows the recombinant DNA molecules as characterized by restriction endonucleases.

FIG.3 illustrating the strategy for insertion of the glucocorticoid receptor site into the recombinant plasmid pMSVHBs4.

FIG.4 shows the recombinant DNA molecules containing the glucocorticoid receptor site as characterized by restriction endonucleases.

FIG.5 illustrating the strategy for construction of recombinant DNA molecules in which the oncogene v-mos$^M$ is deleted from the MSV genome.

FIG.6 shows the recombinant DNA molecules in which the transforming region of MSV is omitted as characterized by restriction endonucleases.

FIG.7 presents data on the production rate of the cell lines disclosed in this invention and their comparison to the human established cell line PLC/PRF/5 (J.J. Alexander, et al., S.Afr.med.J., vol.50, 2124 - 2128 (1976)) which also produces HBsAg, and is considered to be a source for vaccine other than human serum. (A) Cells are kept in maintenance medium. (B) Medium is changed daily.

FIG.8 shows the electrophoretic analysis of immunoprecipitated HBsAg polypeptides. Labelled proteins from e.g. cell line Yl and from untransfected NIH 3T3 cells are incubated with preimmune guinea pig serum or with high-titered anti-HBsAg serum from guinea pigs and precipitated as

0105141

described. Proteins are analyzed by polyacrylamide gel electrophoresis and autoradiography. Lane a, $^{14}$C-labelled protein standards - globulins (150K), bovine serum albumin (68K), ovalbumin (46K), carbonic anhydrase (30K), and lactoglobulin A (18.4K); lanes b through d, proteins from cell line Y1 incubated with 0.1 ul (lane b), 1 ul (lane c), and 10 ul (lane d) of anti-HBsAg serum; lane e, proteins from cell line Y1 incubated with 10 ul of preimmune serum; lane f, proteins from untransfected NIH 3T3 cells incubated with 10 ul of anti-HBsAg serum.

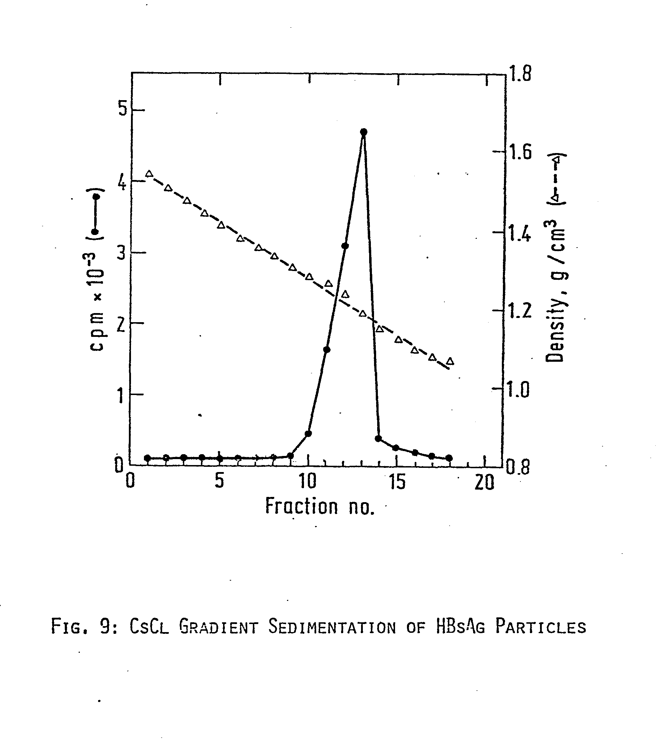

FIG.9 shows as an example the CsCl gradient sedimentation of HBsAg isolated from nutrient medium of cell line Y1.

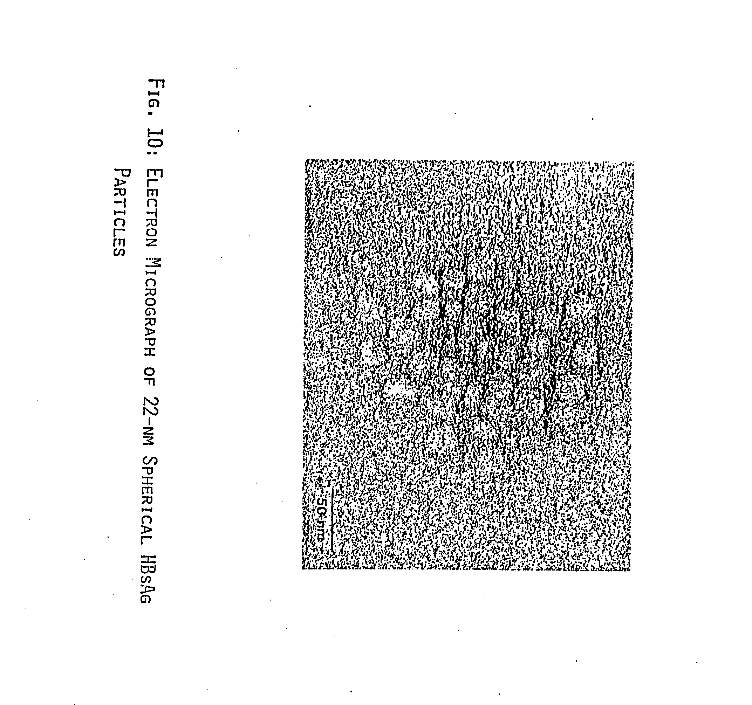

FIG.10 shows as an example an electron micrograph of 22-nm spherical HBsAg particles produced by the cell line Y1. Particles are visualized by negative staining with 1% phosphotungstic acid.

TABLE 1 shows as an example the anti-HBsAg titer obtained in sera of guinea pigs immunized with HBsAg particles from cell line Y1.

DESCRIPTION OF SPECIFIC EMBODIMENTS

In the following the preparation of the starting material for the establishment of novel cell lines producing HBsAg of this invention is described:

I., Construction of eukaryotic vectors for establishing cell cultures of vertebrate origin producing HBsAg:

(a) Preparation of the MSV genome

(b) Preparation of the BPV genome

(c) Preparation of the HBsAg gene

(d) In vitro recombination of MSV (a) and BPV (b), respectively, with HBsAg gene (c) to form novel recombinant plasmids (pMSVHBs4, pMSVHBs9, pBPVHBsR/8) as starting material for the inventive process.

(e) Establishment of cell lines producing HBsAg with the novel recombinant plasmids obtained in (d).

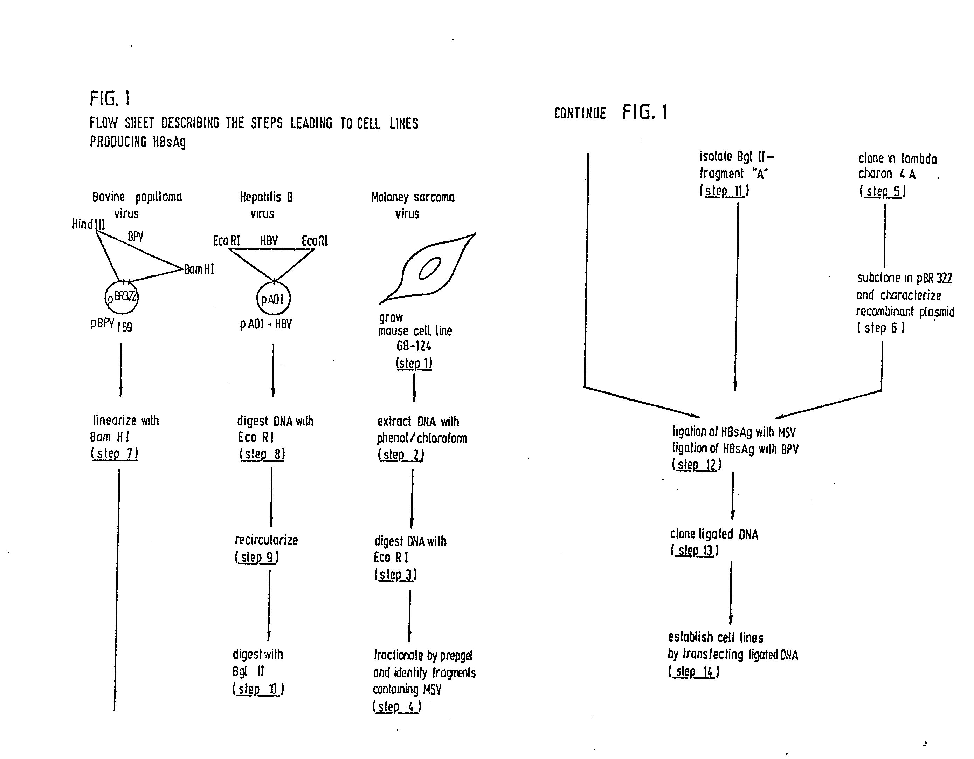

The steps mentioned in the following detailed description of (a) to (e) relate to the flow sheet of Fig. 1.

II., Introduction of an additional eukaryotic expression signal into the genome of MSV:

(f) Preparation of the glucocorticoid receptor site (GRS).

(g) In vitro recombination of pMSVHBs4 (d) with GRS (f) to form novel recombinant plasmids (pM4GRS/LR, pM4GRS/LL, pM4GRS/L2, pM4GRS/R) as starting material for the inventive

process.

(h.) Establishment of cell lines producing HBsAg with the novel recombinant plasmids obtained in (g).

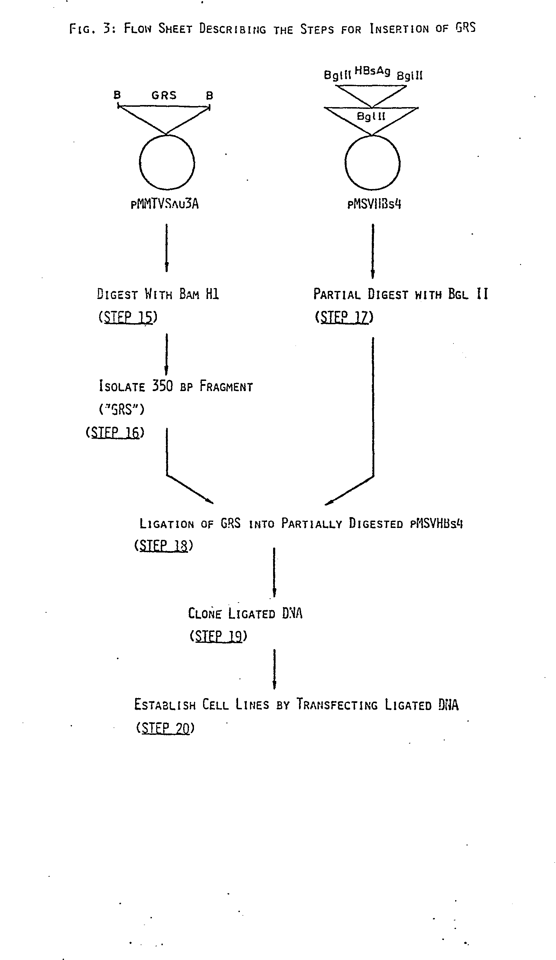

The steps mentioned in the following detailed description of (f) to (h) relate to the flow sheet of Fig. 3.

III., Omission of the transforming region contained in the genome of MSV:

(i) Deletion of the oncogene v-mos$^M$ from MSV genome.

(k) In vitro recombination of the modified MSV genome (i) with HBsAg gene (c) to form novel recombinant plasmids (p2LTRHBs80, p2LTRHBs82) as starting material for the inventive process.

(l) Establishment of cell lines producing HBsAg with the novel recombinant plasmids obtained in (k).

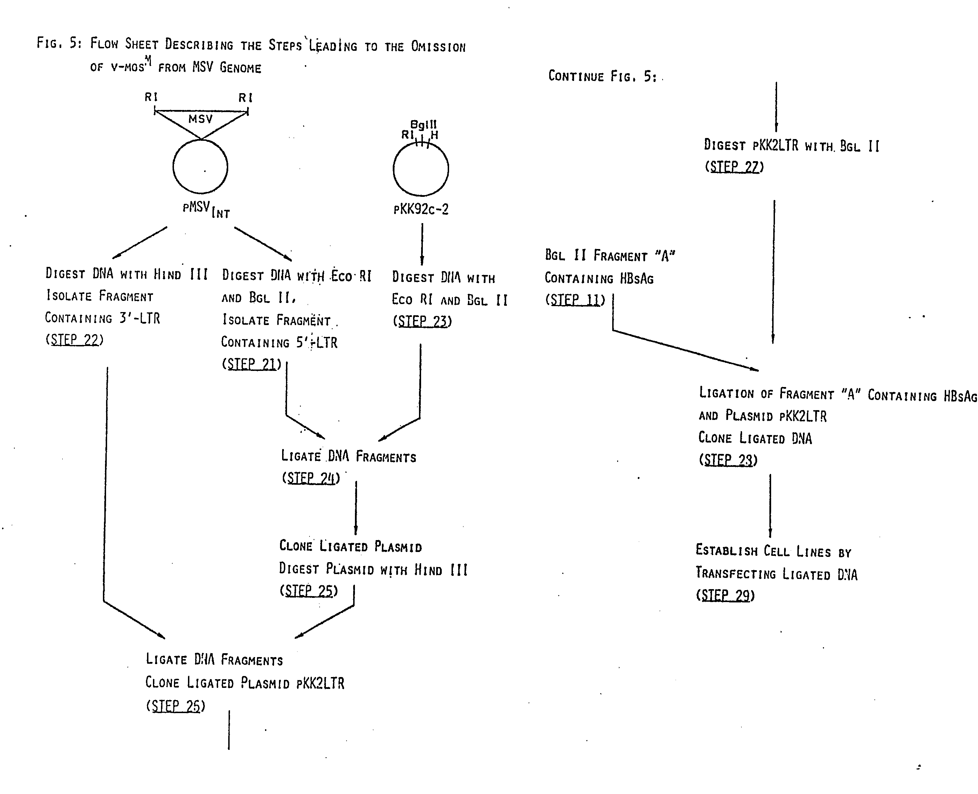

The steps mentioned in the following detailed description of (i) to (l) relate to the flow sheet of Fig. 5.

I., CONSTRUCTION OF EUKARYOTIC VECTORS FOR ESTABLISHING VERTEBRATE CELL CULTURES PRODUCING HBsAg

(a) PREPARATION OF THE MSV GENOME

Step 1: In 1973 a mouse cell line called G8-124 was established and described by Judith Ball, et al., Virology vol.56, 268 (1973). This cell line contains and produces several types of RNA-Tumorviruses, among them MSV. The RNA-Tumorvirus exists within the cell as an integrative part of the cellular genome from which new virions originate. Cells are grown in Dulbecco's modified Eagle's medium supplemented with penicillin at 100 units/ml and streptomycin at 100 ug/ml.

Step 2: The integrated provirus of MSV is isolated from the host cell genome. For this purpose cellular DNA is isolated from $1 \times 10^8$ cells. Cells are detached from the surface of a 140 mm Petri dish by incubation for lo to 15 minutes at room temperature in 5 ml phosphate buffered saline (PBS) containing 10 mM EDTA. Detached cells are harvested by centrifugation at 150 x g for 3 minutes at $4^{\circ}$C in 50 ml Corning tubes. Cell pellet obtained is washed once with cold PBS and centrifuged again with 150 x g for 3 minutes at $4^{\circ}$C. Cells are resuspended in 10 ml of lysisbuffer (10 mM Tris, pH7.4; 1,5 mM $MgCl_2$; 150 mM NaCl) to which the nonionic detergent ethylphenylpolyethyleneglycol (NP-40) is added to a final concentration of 1%, immediately followed by vigorous shaking on a Vortex apparatus for 15 seconds. In this way the outer cell membrane is lysed leaving the nuclear membrane intact. Cell nuclei are pelleted by centrifugation at 750 x g for 2 minutes at $4^{\circ}$C. Supernatant is very carefully taken off since the pellet of the nuclei is rather soft. The following steps are performed at room temperature. Pelleted cell nuclei are resuspended in lo ml of a saline-EDTA solution (10 mM EDTA; 150 mM NaCl) in which the pellet is broken by gentle shaking. Proteinase K (Boehringer Mannheim) is added to a final concentration of 100 - 200 ug / ml and mixed. Sodium dodecyl sulfate (SDS) is added to a final concentration of 0.5% and mixed. The nuclear membranes are dissolved by SDS, DNA is liberated causing a considerable increase in viscosity. The mixture is incubated at 37°C for at least 4 hours up to 24 hours for digestion of proteins. The DNA is extracted by adding an equal volume of phenol, saturated with 10 mM Tris pH 7.8. The mixture is shaken gently for 15 minutes without applying shearing forces. The aqueous and organic phases are separated by centrifugation at about 100 x g. The aqueous phase which contains DNA is transferred with a wide mouth Pasteur pipette to a new 50 ml Corning tube. Phenol extraction is repeated once as described above. An equal volume of chloroform/isoamylalcohol (24:1) is added to the aqueous phase and gently shaken for 5 minutes. The aqueous and organic phases are separated by centrifugation. The aqueous phase is transferred to a new 50 ml Corning tube. Chloroform extraction procedure is repeated once. The aqueous phase is mixed with a 2.5 fold volume of isopropanol in order to precipitate the DNA. DNA is pelleted by centrifugation at 100 x g for 5 minutes, supernatant is decanted carefully. The DNA is resuspended in 5 ml of 10 mM Tris, 200 mM NaCl, pH 7.8 and precipitated once more with 2.5 fold volume of ethanol. The pelleted DNA is dissolved over night in 10 mM Tris, pH 7.8 at room temperature. The final yield following this protocol is in the range of 1000 - 2000 ug DNA starting from 1 x $10^8$ cells.

Step 3: The so prepared DNA from G8-124 mouse cells is cleaved into fragments by restriction endonuclease Eco RI. This enzyme has been shown not to cleave the viral genome (C.van Beveren, et al., Cell, vol.27, 97-108 (1981)). In order to isolate a fragment containing the viral genome 1000 ug of cellular DNA is digested in a total volume of 5000 ul containing 1000 units of Eco RI, 100 mM Tris, 5 mM $MgCl_2$, 50 mM NaCl, 2 mM mercaptoethanol, pH 7.8 at 37°C. The digested DNA is precipitated by adding 200 mM NaCl and 2.5 fold volume of ethanol at -20°C and resuspended in 3000 ul of 10 mM Tris pH 7.8. The digested DNA is fractionated by preparative gel electrophoresis, 0.7% agarose, (Sigma, Type II) in 40 mM Tris Base, 1 mM EDTA, 5 mM Na-acetat, adjusted with glacial acetic acid to pH 7.8; Gel size: 20 cm long x 17 cm wide x 0.7 cm high. Slot size: 13.5 cm long x 0.5 cm wide x 0.7 cm high. Just before loading the DNA solution into the slot of the gel, 800 ul of 40% glycerol solution containing 30 mM EDTA, 0.05% bromophenolblue , 0.1% SDS and 400 ul of 2% agarose at a temperature of about $60^{o}C$ are added to the DNA solution. The cellular DNA fragments are separated by applying 100 Volts during 18 hours.

Step 4: In order to detect and to isolate the DNA fragments, which contain the complete genomic form of integrated MSV, a strip of the preparative gel (20 cm long x 2 cm wide) is cut out for Southern blotting (E.M.Southern, J.Mol.Biol. vol.98, 503-517 (1975)). The rest of the preparative gel is wrapped with cellulose hydrate film (cellophane) and stored at $4^{o}C$ until further processing. The DNA in the cut out strip is denatured by exposing to 366 nm UV light for 10 minutes and by incubation in a solution of 0.5 N NaOH/1 M NaCl for 45 minutes for DNA strand separation. The gel is neutralized by incubating in 0.5M Tris/1.5 M NaCl, pH 7.5 for 60 minutes. The DNA is transferred to a nitrocellulose filter by soaking 3 M NaCl/0.3 M Na citrate (20 X SSC) for 20 hours through the gel, which is covered by the nitrocellulose filter and dry paper towels. Thereafter the nitrocellulose filter is heated for 2 hours under vacuum at $80^{o}C$ which results in covalent linkage of the DNA strands to the nitrocellulose. A radioactive DNA probe homologous to the transforming gene of the MSV (v-mos[M]) is prepared by nicktranslation (P.W.J. Rigby, et al., J Mol.Biol. vol.113, 237 - 251 (1977)) by which the DNA fragments containing integrated MSV can be made visible on X-ray films. For this purpose 0.5 ug of the Xba I/Hind III DNA fragment from molecularly cloned unintegrated MSV encoding the transforming gene product are incubated in a total volume of 30 ul containing 50 mM Tris pH 7.8, 5 mM $MgCl_2$, 10 mM mercaptoethanol, 0.1 mM dATP, 0.1 mM dGTP, 0.1 mM dTTP, 50 uCi [32]PdCTP (spec. act. 400 to 800 Ci/Mol), 10 units DNA polymerase I, 3 ul of a $2 \times 10^{-5}$ fold dilution of 1 mg/ml DNase I for 70 minutes at 13°C ; yielding $3 \times 10^6$ to $12 \times 10^6$ total cpm, i.e. $1 \times 10^7$ to $5 \times 10^7$ cpm/ug DNA. The nitrocellulose filter is sealed in a plastic bag containing 10 ml of the following prehybridization mixture: 50% formamide, 5 x SSC, 50 mM Na-phosphat pH 7.0, 5 x Denhardt's solution (0.1% BSA, 0.1% ficoll, 0.1% polyvinylpyrrolidone), 250 ug/ml denatured calf thymus DNA or salmon sperm DNA. The nitrocellulose filter is incubated for at least 1 hour up to 4 hours at 45°C in this solution. After that the prehybridization mixture is exchanged against the hybridization mixture: 50% formamide, 5 x SSC, 20 mM Na phosphate pH 7.0, 1 x Denhardt's solution (0.02% BSA, 0.02% ficoll, 0.02% polyvinylpyrrolidone), 100 ug/ml denatured calf thymus DNA or salmon sperm DNA, $5 \times 10^5$ cpm/ml radioactive nicktranslated DNA probe. This incubation is followed by extensive washing three times - 5 minutes each - in 2 x SSC, 0.1% SDS at room temperature and twice for 15 minutes in 0.1 x SSC, 0.1% SDS at 50°C. The nitrocellulose filter is dried thoroughly at 60°C for 10 minutes. The dried filter is exposed to two X-ray films XR5 (Kodak) between two intensifying screens. The first X-ray film is developed after 3 days exposure, the second film after 7 days exposure. Four hybridizing DNA-fragments can be seen at about 14 kb, 12 kb, 10 kb, and 8 kb. The X-ray film is aligned to the rest of the preparative gel which is stored at 4°C. The gel slice containing the DNA fragments ligning up with the 8 kb hybridizing band visible on the X-ray film is cut out of the gel. The DNA fragments are eluted electrophoretically from the gel slice. The DNA obtained from that gel slice is extracted by phenol/chloroform as described above.

Step 5: The DNA of bacteriophage lambda Charon 4 A (J.R.de Wet et al., J. of Virology, vol.33, 401-410 (1980)) is prepared for ligation to the DNA fragments obtained from the preparative gel. Lambda Charon 4 A DNA is digested by restriction endonuclease Eco RI. There are three Eco RI sites in this bacteriophage genome: at 19801 bp, 26693 bp and 34524 bp. The total length of the linear bacteriophage genome amounts to 45410 bp. Four fragments are obtained after digestion with Eco RI at about 19 kb, 11 kb, 7.8 kb, and 6.8 kb. The bacteriophage genome between position 19801 bp and 34524 bp is substituted by the Eco RI fragments obtained from the preparative gel. Therefore the 19 kb fragment and 11 kb fragment are isolated from the preparative agarose gel as described above and linked to the 8kb DNA fragments from the G8-124 mouse cell DNA in 15 ul total volume containing 0.01 units of T4 DNA-ligase (BRL), 66 mM Tris pH 7.6, 6.6 mM $MgCl_2$, 10 mM DTT, 0.4 mM ATP. The reaction mixture is incubated at $4^{\circ}C$ for 18 to 24 hours. The recombinant bacteriophage genome of lambda Charon 4 A is transferred into the E.coli strain K801 after in vitro packaging for forming infectious phages. In vitro packaging and screening for recombinant phages is performed following the procedures described in "Methods in Enzymology", Vol. 68, "Recombinant DNA" , Ray Wu ed., Academic Press, New York, London, Toronto, Sydney, San Francisco, chapt. 19 and chapt. 20 (1979).

Bacteriophage lambda Charon 4 A found to be positive for the transforming gene of the RNA-Tumorvirus is isolated from plaques and reinfected on E.coli HB 101 for mass culture from which the phage DNA is obtained. Insert DNA of the recombinant phage DNA containing the integrated MSV genome from the G8-124 mouse cell genome is isolated in a preparative style from agarose gel and subsequently inserted into the Eco RI site of the plasmid vector pBR322. The recombinant plasmid is named $pMSV_{INT}$.

Step 6: This recombinant plasmid $pMSV_{INT}$ is extensively characterized by restriction endonucleases Eco RI, Kpn I, Sal I, Bgl II and Hind III. The restriction sites are found to be in agreement with the sequence of the unintegrated MSV (C.v.Beveren, et al., Cell, vol.27, 97-108 (1981)). The length of the adjacent cellular DNA at the 5'end is about 100 bp and 2200 bp at the 3'end.

0105141

The recombinant plasmid $pMSV_{INT}$ is checked for its biological activity regarding the morphological transformation of NIH 3T3 mouse fibroblasts which is found to be in the range of 1000 to 2000 focus forming units/ug of MSV DNA. After reintroduction of this molecularly cloned MSV genome into NIH 3T3 mouse fibroblasts it is recovered as an infectious particle upon rescue with its helper virus Moloney Leukemia Virus (MLV).

(b) PREPARATION OF THE BPV GENOME

Step 7: The recombinant plasmid pBPV$_{T69}$ containing the transforming fragment of Bovine Papilloma Virus type 1 is described (P.M.Howley, et al., in "Viruses in Naturally Occuring Cancers", M.Essex, H.zur Hausen, G.Todaro eds., Cold Spring Harbor Laboratory, Cold Spring Harbor, NY, vol.7, 233-247 (1980)). This recombinant plasmid DNA is digested with restriction endonuclease Bam HI. Two ug of this DNA are incubated in a total volume of 20 ul containing 4 units Bam HI, 150 mM NaCl, 6mM Tris pH 7.8, 6mM MgCl$_2$ for 1 hour at 37$^O$C.

(c) PREPARATION OF THE HBsAg GENE

Step 8: The HBV genome molecularly cloned is described by I.W.Cummings et al., Proc.Natl.Acad.Sci.U.S.A., vol.77, 1842-1846 (1980) who purified viral particles from serum of a RED CROSS donor positive for the HBsAg, subtype ad/w (AMERICAN RED CROSS, SAMPLE ARC 2375). The EcoRI linear HBV genome has been cloned by these authors in bacteriophage lambda gtWES and subcloned in bacterial plasmid pAO1.

The HBV genome from this recombinant plasmid pAO1-HBV is isolated by digestion with restriction endonuclease Eco RI followed by size fractionation on preparative agarose gel as described above.

Step 9: The linear Eco RI fragment representing the HBV genome is recircularized by ligation in order to overcome the permutation caused by molecular cloning and to restore a functional genomic organization. The religation is carried out as follows: 2 ug of Eco RI linear HBV genome is

incubated in a total volume of 200 ul with 1 unit T4 DNA ligase, 60 mM Tris pH 8.0, 6 mM $MgCl_2$, 10 mM DTT, 0.4 mM ATP. This represents a highly diluted solution in order to favor intramolecular ligation. Alternatively permutation can be overcome by head-to-tail construction of the linear Eco RI HBV genome.

Step 10: One ug of recircularized HBV-DNA is digested with 4 units of restriction endonuclease Bgl II in 6.7 mM Tris pH 7.8, 6.7 mM $MgCl_2$ and 6.7 mM mercaptoethanol at $37^O$C. The digestion mixture contains two HBV DNA fragments, i.e. one fragment "A" encoding the structural gene for HBsAg, its viral promoter, its leader sequence and its polyadenylation signal besides two residual fragments of the structural gene for the core antigen (HBcAg) at the 5'end and 3'end of this fragment and another fragment "B" encoding an incomplete structural HBcAg gene.

Step 11: The digestion mixture obtained is used directly for in vitro recombination with the MSV genome described above under (a) and the BPV genome, respectively, as described above under (b). However, the in vitro recombination can be performed also with the selected and isolated fragment "A".

(d) IN VITRO RECOMBINATION OF MSV AND BPV RESPECTIVELY WITH THE HBsAg GENE (inventive process)

Step 12: Two ug of circular plasmid $pMSV_{INT}$ are linearized with restriction endonuclease Bgl II in 6.7 mM Tris pH7.8, 6.7 mM $MgCl_2$ and 6.7 mM mercaptoethanol at $37^O$C. In order to prevent the ligation of linear $pMSV_{INT}$ by itself the phosphate group is removed from the 5'end by alkaline phosphatase (CIAP, Boehringer Mannheim). The linearized pMSVINT is precipitated by adding 200 mM NaCl and a 2.5 fold volume of ethanol. The pelleted DNA is resuspended in a total volume of 200 ul containing 5 mM Tris pH 9.5 and 1 unit of alkaline phosphatase and incubated at $60^{\circ}C$ for 30 minutes. The incubation is followed by phenol extraction which is performed twice. The aqueous phase is taken and mixed with 1 ug of Bgl II fragments of the HBV genome described above. The mixture is ethanol precipitated and resuspended in 20 ul of ligation buffer (6 mM Tris pH 7.6, 6 mM $MgCl_2$, 10 mM DTT, 0.4 mM ATP, 0.5 units T4 DNA ligase) and incubated for 30 minutes at $15^{\circ}C$. After that time the mixture is diluted by ligation buffer up to 200 ul and another 0.5 units of T4 DNA ligase is added and incubated for 15 hours at $4^{\circ}C$.

The known bacterial strain E. coli HB 101 is made competent for the reintroduction of the plasmid pMSV$_{INT}$ recombined with the Bgl II fragments of the HBV genome. A bacterial culture is grown to a density of $OD_{650}$ = 0.8. From this culture 20 ml are taken and bacteria are sedimented by centrifugation. The pelleted bacteria are resuspended in 10 ml 50 mM $CaCl_2$, sedimented again and resuspended in 2 ml 50 mM $CaCl_2$ and incubated for 30 minutes on ice. For introducing the recombined pMSV$_{INT}$ into E.coli 100 ul of this bacterial suspension are mixed with 100 ul buffer (10 mM Tris pH 7.6, 10 mM $MgCl_2$, 10 mM $CaCl_2$) and 40 ul from the ligation assay of pMSV$_{INT}$ and Bgl II fragments of the HBV genome described above. This mixture is incubated for 1 hour on ice, followed by an incubation at $42^{\circ}C$ for 1 minute and another incubation at $20^{\circ}C$ for 10 minutes, after which 1 ml of LB medium (5g NaCl, 10g Bactotryptone/Difco, 5g yeast extract/Difco in 1 liter destilled water) is added and the mixture is shaken for 30 minutes at $37^{\circ}C$. After this the mixture is distributed in 300 ul portions per agar plate containing 50 ug/ml ampicillin. Several bacterial colonies are isolated from these agar plates. These colonies are checked for the presence of the recombinant plasmid. Minipreparations for plasmid DNA are performed according to H.C.Birnboim and J. Doly, Nucl.Acids Res., vol.7, 1513-1523,

0105141

(1979). Of course, other bacterial hosts may be used for propagating the recombinant plasmid DNA, e.g. E.coli C600 or AB301.

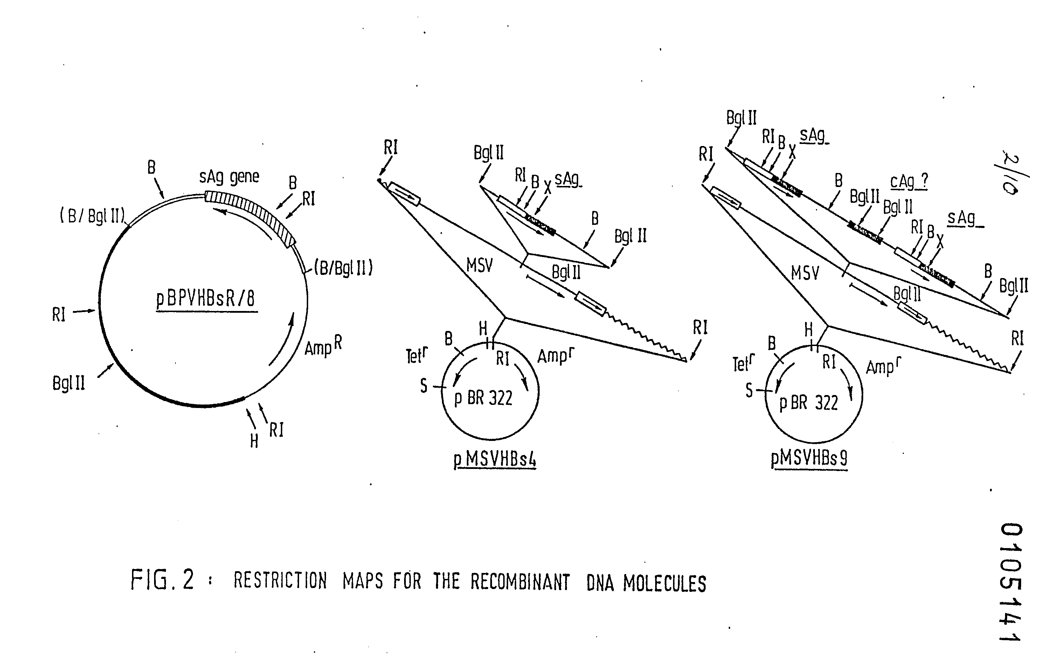

Step 13: Several bacterial colonies are found to carry the recombinant plasmid. Plasmids are isolated in a preparative style and characterized. Two of the plasmid clones are identified to contain MSV and the HBsAg gene. These plasmids are named: pMSVHBs4 and pMSVHBs9. These recombinant plasmids are characterized with various restriction endonucleases. Their structures are shown in Fig.2.

The in vitro recombination of the BPV genome obtained under b) with the HBsAg gene prepared under c) is carried out completely analogous as described for the recombination of the MSV genome with the HBsAg gene. This recombinant plasmid is named : pBPVHBsR/8.

The three recombinant plasmids described above are introduced into competent E.coli, strain HB 101. The transformants obtained, HB101-J1, HB101-J2 and HB101-Y1, have been deposited with the "Deutsche Sammlung von Mikroorganismen" (DMS), Goettingen, FRG, and received the accession numbers 2463, 2464, and 2465 respectively.

(e) ESTABLISHMENT OF CELL LINES PRODUCING HBsAg WITH THE NOVEL RECOMBINANT PLASMIDS OBTAINED IN (d) (inventive process)

Step 14: The recombinant plasmids pBPVHBsR/8, pMSVHBs4 and pMSVHBs9, respectively, are introduced into NIH 3T3 mouse fibroblasts by transfection procedures described by J.Doehmer, et al., Proc.Natl.Acad.Sci.U.S.A., vol.79, 2268-2272 (1982). Mouse fibroblasts which take up and maintain the plasmid DNA are transformed morphologically by the oncogene of MSV or the transforming region of BPV, respectively, into spindle shaped form. These cells are overgrowing the untransformed cells in a characteristic manner as a second layer. These cells are picked with a cloning cylinder and propagated for mass culture in a conventional manner in a conventional nutrient medium (Dulbecco's modified Eagle's medium, supplemented with 10% calf serum and 50 ug kanamycin/ml). In order to remove the untransformed cells which are picked together with the transformed cells limiting dilutions are performed to clone out the transformed cells free of untransformed cells. Alternatively, growth in soft agar was used as a selection criterion for transformed cells.

Three of these clones are established as cell lines: Y1, established after transfection with the recombinant plasmid pBPVHBsR/8, J1 and J2, established after transfection with the recombinant plasmids pMSVHBs4 and pMSVHBs9, respectively. These cell lines are shown to produce and release HBsAg into the culture medium. HBsAg is detected by radioimmunoassay (AUSRIA II, Abbott Laboratories).

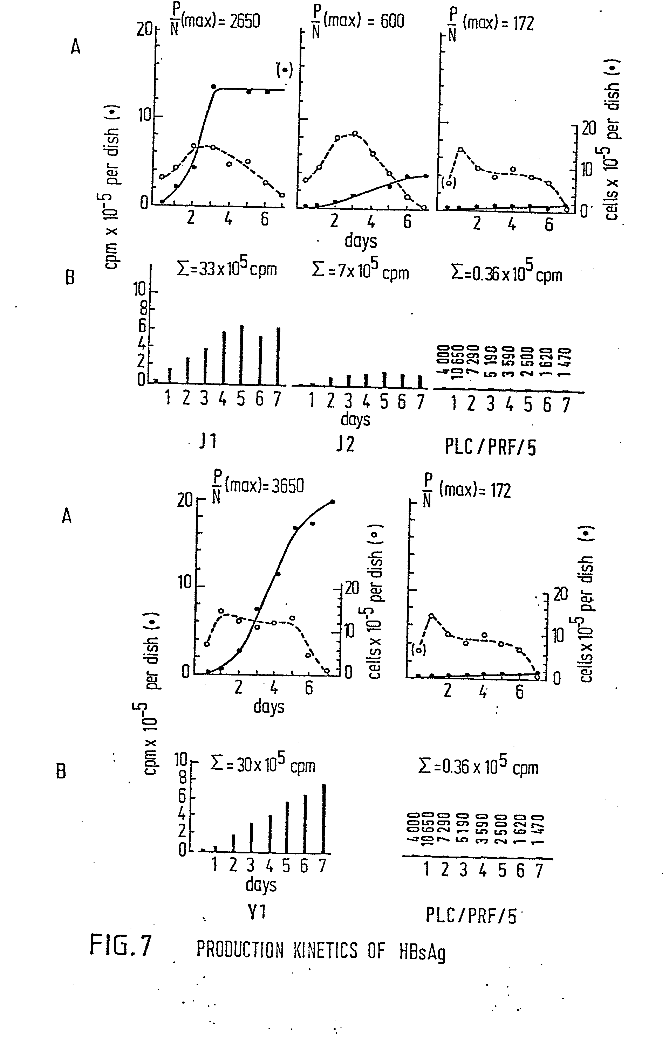

Kinetic studies on the production of HBsAg (Fig. 7) by these cell lines are performed and compared with the known human hepatoma cell line (PLC/PRF/5), mentioned above. Cell lines Y1 and J1 produce up to 400 ng HBsAg/ml of culture medium/day. J2 produces 60 ng HBsAg/ ml of culture medium/day. Under the same conditions described in Fig.7 the human hepatoma cell line PLC/PRF/5 produces 10 ng HBsAg/ ml of culture medium/day. These results are easily reproducible.

The identity of HBsAg produced by the cell lines Y1, J1 and J2 is confirmed by
(i) SDS polyacrylamide gel-electrophoresis after immunoprecipitation of $^{35}$S-cysteine labelled HBsAg,

(ii) CsCl density gradient,

(iii) electron microscopy and

(iv) immunogenicity studies in guinea pigs.

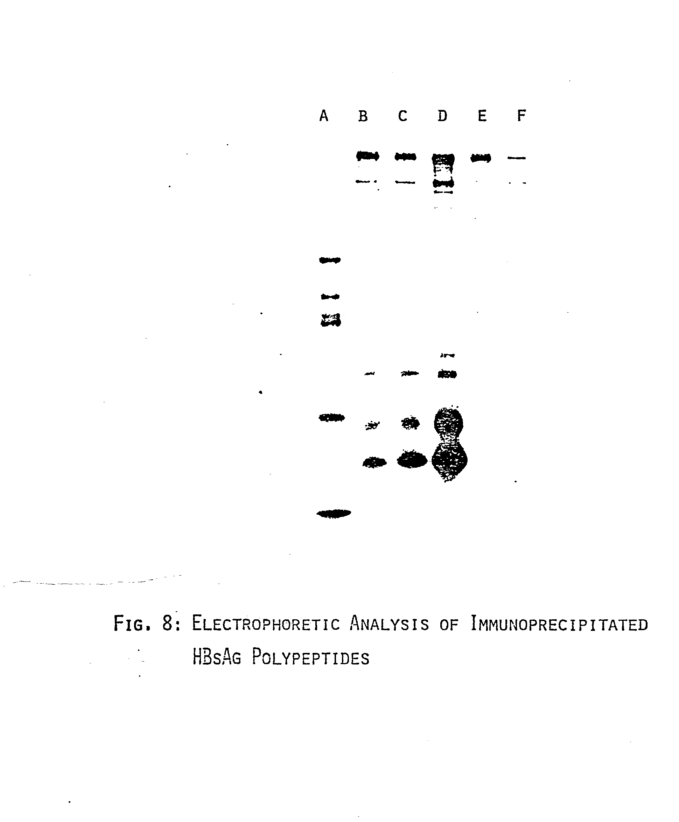

(i) In order to determine the polypeptide composition cells are biosynthetically labelled with 400 uCi of $^{35}$S-cysteine (New England Nuclear Corp.). After overnight incubation at $37^{O}$C medium is collected and proteins are incubated with anti-HBsAg serum from guinea pigs or from humans. Immunoprecipitation and analysis by SDS polyacrylamide gel-electrophoresis is done as described by W. Stibbe and W. Gerlich, Virology, vol. 123, 436 - 442 (1982). As shown by Stibbe and Gerlich for HBsAg from human serum four polypeptides are found with molecular weights of about 24 K, 28 K, 34 K, and 37 K (see Fig. 8). The major components are the 24 K and the 28 K forms. The appearance of the 28 K form indicates glycosylation of the 24 K form of HBsAg; see D.L. Peterson, The Journal of Biological Chemistry, vol. 256, 6975 - 6983 (1981).

(ii) For buoyant density determination HBsAg is isolated and purified by low speed centrifugation (1000 x g) of the supernatant culture medium in order to remove cell debris. Thereafter HBsAg is pelleted by high speed centrifugation (10000 x g) and resuspended in 10 mM Tris pH 7.5, 150 mM NaCl, 1mM EDTA. CsCl is added up to a density of 1.2 g/cm$^3$ and the sample is centrifuged at 235000 x g for 60 hours at $4^{O}$C. Fractions are collected and aliquots are tested for HBsAg by radioimmunoassay after proper dilution. In the CsCl gradient shown in Fig. 9 the HBsAg is present in a fraction corresponding to a density of 1.2 g/ml which is identical to the value found for the 22 nm particles in human serum (L.Gerin, et al., J.Virol. vol.4, 763-768 (1969).

(iii) After concentrating the peak fractions of the CsCl gradient, the particles are further characterized by electron microscopy. As shown in Fig. 10 these particles have a mean diameter of 22 nm and they are identical to HBsAg particles found in human serum.

(iv) HBsAg produced by cell lines Yl, Jl and J2 is immunogenic in guinea pigs. Four guinea pigs are injected subcutaneously with 1 ml physiological saline containing 20 ug HBsAg and 0.1% aluminum hydroxide. After one week the vaccination is repeated. At various times after inoculation blood samples are taken and analysed by radioimmunoassay (AUSAB, Abbott Laboratories). As summarized in Table 1 increasing antibody titers are observed in all guinea pigs after HBsAg injection.

This activity establishes the feasibility of compositions and methods using the HBsAg produced by cultivated animal cells, e.g. transformed NIH 3T3 mouse fibroblasts, LTK⁻ mouse fibroblasts, and monkey kidney cells (Vero cell line) according to the invention for the stimulation of antibody formation and for detection of HBV infections in humans.

The HBsAg produced according to the present invention may be employed alone or with conventional pharmaceutically acceptable carriers or diluents such as saline solutions or additives known in the art such as thiomersal or aluminum hydroxide (0.1%) as adjuvant in the form of a aluminum hydroxide suspension pH 6.7 and mannitol for use in pharmaceutical compositions and methods for the prevention of HBV infection in humans. The vaccine is given three to four times at intervals of 1, 2, 6 and 12 months with doses in the range of 5 to 20 ug HBsAg.

Several cell culture techniques are known in order to scale up for large preparations, e.g. growth on beads or fibers or roller bottles; see e.g. J.Feder and W.R.Tolbert, Sci.Amer., vol.248(1), 24 -31 (1983).

II., INTRODUCTION OF AN ADDITIONAL EUKARYOTIC EXPRESSION SIGNAL INTO THE GENOME OF MSV

(f) PREPARATION OF THE GLUCOCORTICOID RECEPTOR SITE (GRS)

Step 15: Five ug of plasmid pMMTVSau3A, containing the large terminal repeat of mouse mammary tumor virus (MMTV), is digested with Bam H1 as described in step 7. This releases a 4362 bp fragment containing pBR322 and a 350 bp fragment cloned from the MMTV large terminal repeat (J.E.Majors and H.E.Varmus, Nature, vol.289, 253 - 258 (1981)). This 350 bp DNA fragment contains a sequence which binds specifically to the activated glucocorticoid hormone - receptor complex and induces increased transcription rates on adjacent genes.

Step 16: The 350 bp DNA fragment is recovered by preparative gel electrophoresis as described in steps 3 and 4.

(g) IN VITRO RECOMBINATION OF pMSVHBs4 WITH GRS (inventive process)

Step 17: Five ug of plasmid pMSVHBs4 is partially digested with 3 units of the restriction endonuclease Bgl II in 6.7 mM Tris, pH 7.8, 6.7 mM $MgCl_2$ and 6.7 mM mercaptoethanol at $37^{\circ}C$ for 10 minutes. The fragments are separated on a preparative agarose gel. The band representing the complete plasmid pMSVHBs4 linearized by cleavage of one of the two Bgl II sites is recovered by preparative gel electrophoresis as described in steps 3 and 4.
The linear plasmid DNA pMSVHBs4 recovered is recombined in vitro with the 350 bp Bam H1 fragment containing the glucocorticoid receptor site. In order to prevent self-ligation pMSVHBs4 is digested with calf intestine alkaline phosphatase (CIAP) as descibed in step 12.

Step 18: One ug of linearized plasmid pMSVHBs4 is combined with 0.5 ug of the 350 bp Bam Hl fragment from plasmid pMMTVSau3A and ligated as described in step 12. This ligated DNA is introduced into E.coli HB 101.

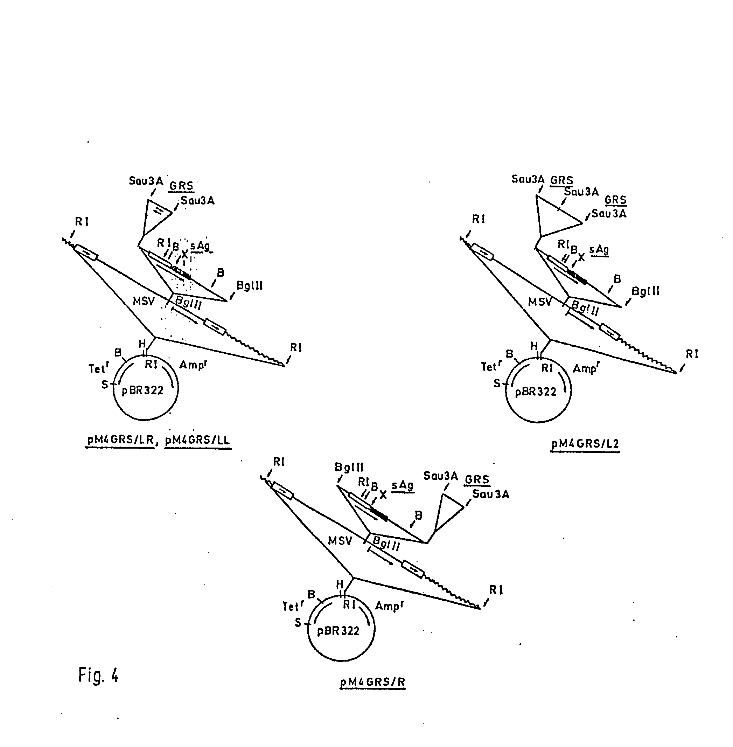

Step 19: Several bacterial colonies are found which contain recombinant plasmids. These plasmids are isolated in a preparative style and characterized by restriction analysis. Two plasmids are found to contain single copies of the 350 bp GRS fragment inserted in the Bgl II site of the 5'end of the HBsAg gene. One of these plasmids, pM4GRSLR, contains the GRS fragment in the same orientation as the HBsAg gene, the other, pM4GRSLL, contains the GRS fragment in the opposite orientation. One plasmid is found which contains two copies of the GRS fragment in the 5' Bgl II site, plasmid pM4GRSL2. A fourth plasmid is found to contain a single copy of the GRS fragment in the Bgl II site 3' of the HBsAg gene. This plasmid is named pM4GRSR. The structures of these plasmids are shown in Fig.4.

(h) ESTABLISHMENT OF CELL LINES PRODUCING HBsAg INDUCIBLE BY GLUCOCORTICOIDS (inventive process)

Step 20: Recombinant plasmids described under step 19 are introduced into NIH 3T3 mouse fibroblasts as described in step 14, as well as into LTK⁻ mouse fibroblasts using the thymidine kinase gene from herpes simplex virus as selective marker (M.Wigler, et al., Cell, vol.16, 777 - 785 (1979)).

III., OMISSION OF THE TRANSFORMING REGION CONTAINED IN THE GENOME OF MSV

(i) DELETION OF THE ONCOGENE v-mos$^M$ FROM MSV GENOME (inventive process)

Step 21: Five ug of plasmid pMSV$_{INT}$ is digested with Eco RI and Bgl II. The 3800 bp fragment containing the left half of the MSV genome, including the 5'-LTR, is recovered by a preparative gel electrophoresis and electroeluted as described in steps 3 and 4.

Step 22: Five ug of plasmid pMSV$_{INT}$ is digested with Hind III and the 1000 bp fragment containing the 3'-LTR of MSV is isolated by gel electrophoresis and electroelution as described in steps 3 and 4.

Step 23: The cloning plasmid pKK92c-2 is digested with the two restriction enzymes Eco RI and Bgl II.

Step 24: The 3800 bp fragment of pMSV$_{INT}$ is ligated into the linearized plasmid obtained in step 23 and transformed into E.coli HB101. The asymetric restriction sites ensure that all recombinant plasmids contain the MSV 5'-LTR in the same orientation.

Step 25: This plasmid is isolated in a preparative style and digested with restriction endonuclease Hind III.

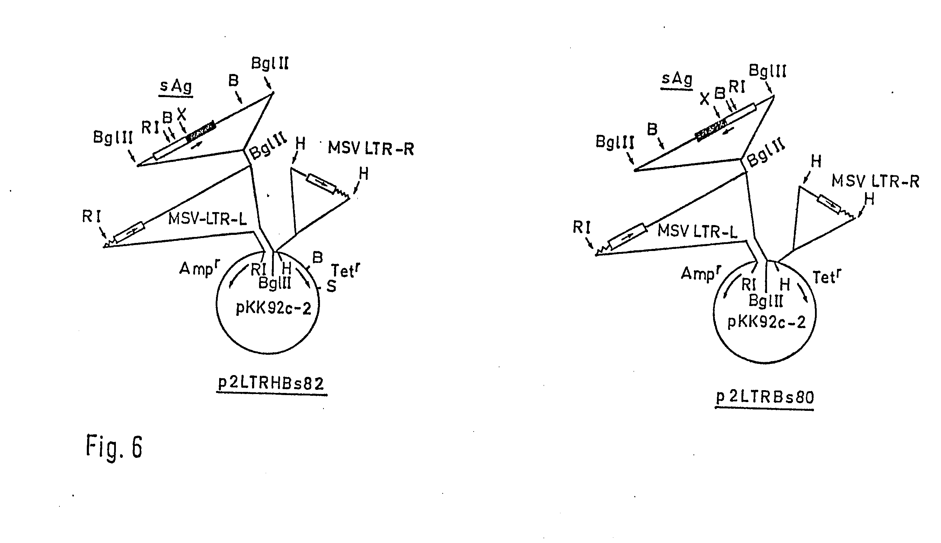

Step 26: The 1000 bp Hind III fragment from pMSV$_{INT}$ is ligated into this site and transformed into E.coli HB101. Bacterial colonies shown to contain recombinant plasmids are characterized by restriction endonuclease digestion with Kpn I in order to identify plasmids containing the two MSV LTRs in the same orientation. One recombinant plasmid, pKK2LTR, is isolated in a preparative style and characterized in detail by restriction endonuclease analysis; see Fig.6.

(k) IN VITRO RECOMBINATION OF MODIFIED MSV GENOME   (pKK2LTR)
WITH THE HBsAg GENE (inventive process)

Step 27: Plasmid pKK2LTR obtained in step 26  is   linearized
with restriction endonuclease Bgl II.

Step 28: The linearized plasmid pKK2LTR is  ligated   to   the
Bgl II fragment of HBV containing the HBsAg  gene  (isolated
as described in step 11) and transformed into E.coli  HB101.
Bacterial  colonies  containing  recombinant  plasmids    are
isolated  and  characterized  by  restriction   endonuclease
digestion according to step 6 to determine  the   orientation
of the HBsAg gene. One  plasmid,  p2LTRHBs82,  is  found  to
contain the HBsAg gene in the same orientation  as   the   two
LTRs. Another plasmid, p2LTRHBs80 is found  to   contain   the
HBsAg gene in the opposite orientation. These  two  plasmids
are shown in Fig.6.

(1) ESTABLISHMENT OF CELL LINES PRODUCING HBsAg WITH THE NOVEL RECOMBINANT PLASMIDS OBTAINED IN (k) (inventive process)

Step 29: Recombinant plasmids described in step 28 are introduced into NIH 3T3 mouse fibroblasts as well as Vero cells by microinjection together with the bacterial gene xanthine - guanine phosphoribosyltransferase (gpt) as selective marker (R.C.Mulligan and P.Berg, Proc.Natl.Acad. Sci.U.S.A., vol.78, 2072 - 2076 (1981)).

CLAIMS:

1. A recombinant DNA molecule comprising

a) a DNA fragment of Bovine Papilloma Virus (BPV) containing the transforming region and the origin of replication of the genome and

b) A DNA fragment of Hepatitis B Virus containing the structural gene of the Hepatitis B surface antigen, a eukaryotic promoter, leader sequence and polyadenylation signal.

2. A recombinant DNA molecule comprising

a) the integrated proviral DNA of Moloney Mouse Sarcoma Virus (MSV) containing the complete viral genome including the large terminal repeats (LTR) and the transforming gene (v-mos$^M$) and

b) A DNA fragment of Hepatitis B Virus containing the structural gene of Hepatitis B surface antigen, a eukaryotic promoter, leader sequence and polyadenylation signal.

3. A recombinant DNA molecule according to claim 1 or 2, wherein the promoter, leader sequence, and polyadenylation signal is of Hepatitis B Virus origin.

4. A recombinant DNA molecule according to any one of claims 1 to 3 comprising additional sequences enhancing the transcriptional rate of the structural gene of Hepatitis B surface antigen.

5. A recombinant DNA molecule according to any one of claims 1 to 4, comprising additionally a fragment of the large terminal repeat (LTR) from mouse mammary tumor virus (MMTV) containing the glucocorticoid receptor site (GRS).

6. A recombinant DNA molecule according to any one of claims 2 to 5, wherein the proviral DNA a) is an integrated proviral DNA of Moloney Mouse Sarcoma Virus obtained from the genome of mouse cell line G8-124.

7. A recombinant DNA molecule according to any one of claims 1 to 6 wherein component a) does not contain the transforming region.

8. A recombinant DNA molecule according each of claims 1 to 7 comprising additionally a cloning vehicle capable of replication in a microbial host.

9. A microbial host containing at least one recombinant DNA molecule according to claim 8.

10. Vertebrate cells containing at least one recombinant DNA molecule according to any one of claims 1 to 8 capable of producing in a nutrient medium suitable for propagating said cells at least one Hepatitis B surface antigen stimulating the production of antibodies to Hepatitis B Virus.

11. The use of vertebrate cells according to claim 10 for producing in a nutrient medium suitable for propagating said cells at least one Hepatitis B surface antigen stimulating the production of antibodies to Hepatitis B Virus.

12. Antigen displaying Hepatitis B surface antigenicity and stimulating the production of antibodies to Hepatitis B Virus produced by vertebrate cells according to claim 10.

13. NIH 3T3 mouse fibroblasts containing at least one recombinant DNA molecule according to any one of claims 1 to 8 capable of producing in a nutrient medium suitable for propagating said fibroblasts at least one Hepatitis B surface antigen stimulating the production of antibodies to Hepatitis B Virus.

14. The use of the NIH 3T3 mouse fibroblasts according to claim 13 for producing in a nutrient medium suitable for propagating said fibroblasts at least one Hepatitis B surface antigen stimulating the production of antibodies to Hepatitis B Virus.

15. Antigen displaying Hepatitis B surface antigenicity and stimulating the production of antibodies to Hepatitis B Virus produced by NIH 3T3 mouse fibroblasts according to claim 13.

16. African Green Monkey Kidney cells (Vero cell line) containing at least one recombinant DNA molecule according to any one of claims 1 to 8 capable of producing in a nutrient medium suitable for propagating said cells at least one Hepatitis B surface antigen stimulating the production of antibodies to Hepatitis B Virus.

17. The use of African Green Monkey Kidney cells (Vero cell line) according to claim 16 for producing in a nutrient medium suitable for propagating said cells at least one Hepatitis B surface antigen stimulating the production of antibodies to Hepatitis B Virus.

18. Antigen displaying Hepatitis B surface antigenicity and stimulating the production of antibodies to Hepatitis B Virus produced by African Green Monkey Kidney cells (Vero cell line) according to claim 16.

19. LTK⁻ mouse fibroblasts containing at least one recombinant DNA molecule according to any one of claims 1 to 8 capable of producing in a nutrient medium suitable for propagating said fibroblasts at least one Hepatitis B surface antigen stimulating the production of antibodies to Hepatitis B Virus.

20. The use of LTK⁻ mouse fibroblasts according to claim 19 for producing in a nutrient medium suitable for propagating said fibroblasts at least one Hepatitis B surface antigen stimulating the production of antibodies to Hepatitis B Virus.

21. Antigen displaying Hepatitis B surface antigenicity and stimulating the production of antibodies to Hepatitis B Virus produced by fibroblasts according to claim 19.

22. A pharmaceutical composition for stimulating the production of antibodies in humans to Hepatitis B Virus infection containing at least one antigen displaying Hepatitis B surface antigenicity produced by vertebrate cells according to any one of claims 10, 13, 16 and 19 and a pharmaceutically acceptable carrier or diluent.

23. A diagnostic composition for the detection of antibodies to Hepatitis B Virus containing at least one antigen displaying Hepatitis B surface antigenicity produced by vertebrate cells according to any one of claims 10, 13, 16 and 19.

# FIG. 1

FLOW SHEET DESCRIBING THE STEPS LEADING TO CELL LINES
PRODUCING HBsAg

Bovine papilloma
virus

Hepatitis B
virus

Moloney sarcoma
virus

pBPV T69

pAO1 - HBV

grow
mouse cell line
G8-124
(step 1)

linearize with
Bam HI
(step 7)

digest DNA with
Eco RI
(step 8)

extract DNA with
phenol/chloroform
(step 2)

recircularize
(step 9)

digest DNA with
Eco RI
(step 3)

digest with
Bgl II
(step 10)

fractionate by prepgel
and identify fragments
containing MSV
(step 4)

CONTINUE  FIG. 1

isolate Bgl II—
fragment "A"
(step 11)

clone in lambda
charon 4 A
(step 5)

subclone in pBR 322
and characterize
recombinant plasmid
(step 6)

ligation of HBsAg with MSV
ligation of HBsAg with BPV
(step 12)

clone ligated DNA
(step 13)

establish cell lines
by transfecting ligated DNA
(step 14)

1/10

0105141

FIG. 2 : RESTRICTION MAPS FOR THE RECOMBINANT DNA MOLECULES

FIG. 3: FLOW SHEET DESCRIBING THE STEPS FOR INSERTION OF GRS

```
        B     GRS    B              Bgl II  HBsAg  Bgl II
        └──────┬──────┘              └────────┬────────┘
               │                           Bgl II
              ◯                              ◯
         pMMTVSAu3A                      pMSVHBs4
               │                              │
               ▼                              ▼
```

DIGEST WITH BAM H1             PARTIAL DIGEST WITH BGL II
(STEP 15)                      (STEP 17)

ISOLATE 350 BP FRAGMENT
("GRS")
(STEP 16)

LIGATION OF GRS INTO PARTIALLY DIGESTED pMSVHBs4
(STEP 18)

CLONE LIGATED DNA
(STEP 19)

ESTABLISH CELL LINES BY TRANSFECTING LIGATED DNA
(STEP 20)

Fig. 4

FIG. 5: FLOW SHEET DESCRIBING THE STEPS LEADING TO THE OMISSION
OF V-MOS$^M$ FROM MSV GENOME

RI                    RI

MSV

pMSV$_{INT}$

Bglll
RI H

pKK92c-2

CONTINUE FIG. 5:

DIGEST pKK2LTR WITH BGL II
(STEP 27)

DIGEST DNA WITH HIND III
ISOLATE FRAGMENT
CONTAINING 3'-LTR
(STEP 22)

DIGEST DNA WITH ECO RI
AND BGL II,
ISOLATE FRAGMENT
CONTAINING 5'-LTR
(STEP 21)

DIGEST DNA WITH
ECO RI AND BGL II
(STEP 23)

BGL II FRAGMENT "A"
CONTAINING HBsAg
(STEP 11)

LIGATE DNA FRAGMENTS
(STEP 24)

LIGATION OF FRAGMENT "A" CONTAINING HBsAg
AND PLASMID pKK2LTR
CLONE LIGATED DNA
(STEP 23)

CLONE LIGATED PLASMID
DIGEST PLASMID WITH HIND III
(STEP 25)

ESTABLISH CELL LINES BY
TRANSFECTING LIGATED DNA
(STEP 29)

LIGATE DNA FRAGMENTS
CLONE LIGATED PLASMID pKK2LTR
(STEP 26)

Fig. 6

p2LTRHBs82

p 2LTRBs80

FIG.7 PRODUCTION KINETICS OF HBsAg

FIG. 8: ELECTROPHORETIC ANALYSIS OF IMMUNOPRECIPITATED HBsAg POLYPEPTIDES

FIG. 9: CsCl GRADIENT SEDIMENTATION OF HBsAg PARTICLES

FIG. 10: ELECTRON MICROGRAPH OF 22-NM SPHERICAL HBsAg
PARTICLES